# EUROPEAN PATENT SPECIFICATION

(11) **EP 3 659 917 B1**
(45) Date of publication and mention of the grant of the patent: **29.12.2021**
(21) Application number: 19212170.5
(22) Date of filing: 28.11.2019
(51) Int. Cl.: B64D 11/00, A62B 7/14, A61M 16/06, B64D 13/06

(54) **OXYGEN SYSTEM WITH ELECTRONIC FLOW INDICATION**
SAUERSTOFFSYSTEM MIT ELEKTRONISCHER DURCHFLUSSANZEIGE
SYSTÈME D'OXYGÈNE AVEC INDICATION ÉLECTRONIQUE DU DÉBIT

(30) Priority: 28.11.2018 US 201862772212 P; 25.11.2019 US 201916694703
(43) Date of publication of application: 03.06.2020
(73) Proprietor: Zodiac Cabin Controls GmbH, 22547 Hamburg (DE)
(72) Inventor: Tomasena, Etienne, D-23552 Lübeck (DE); Meckes, Rüdiger, D-23919 Berkenthin (DE); Hollm, Marco, D-25524 Oelixdorf (DE); Westphal, Andreas, D-23701 Eutin (DE)
(74) Representative: Stevenson-Hill, Jack Patrick

(56) References cited:
- EP-A1- 2 371 411
- WO-A1-2016/054097
- US-A1- 2016 325 123
- US-B2- 7 298 280

## Description

### CROSS REFERENCE TO RELATED APPLICATIONS

This application claims the benefit of U.S. Provisional Application No. 62/772,212, filed November 28, 2018.

### FIELD OF THE INVENTION

The present invention relates generally to an oxygen system with electronic flow indication.

### BACKGROUND OF THE INVENTION

On a typical passenger aircraft, the passenger oxygen system includes a face piece (which comes in contact with the passenger face), a valve plate, a breathing bag, a hose connecting the oxygen supply to the face piece, a flow indicator, a strap for the adjustment of the mask to the passenger and an oxygen source. The passenger oxygen mask is connected to an oxygen source which provides oxygen to each passenger during a decompression event on the aircraft. Passenger oxygen masks are stored within a compartment and connected to the oxygen supply. State of the art passenger oxygen systems have a flow indicator connected either to the breathing bag or to the hose. The flow indicator is a small device and therefore its visibility is limited. In absence of light in the aircraft, the status of the flow indicator cannot be seen. In addition this means that the cabin crew cannot see the status of each individual passenger's flow indicator from the cabin crew member's seat position. Rather, the cabin crew member has to walk through the cabin and check the flow indicator of each passenger in case of an aircraft decompression event. This operation is not efficient and the cabin crew cannot focus on a passenger who may need additional oxygen. The present invention provides a passenger oxygen system with a visible flow indicator on the oxygen hose, on the passenger service unit and/or on a central display available to the cabin crew from their seated position.

US2016325123 discloses an aircraft emergency oxygen delivery system; power is generated by the flow of gas over a transducer disposed inside an oxygen delivery tube. A pressure differential gives rise to a temperature difference across the transducer, and the temperature difference can be converted to a voltage. The power thus generated may be used to operate LED indicators visible from the exterior of the tube, a variety of sensors, and wireless communication with a central control system.

US2005011282 describes a fluid flow indication apparatus provided for monitoring and giving an indication of fluid flow.

EP2371411 discloses a visual status indicator for providing a status indication of a fluid transported through a patient circuit between a medical device and a patient, the visual status indicator comprising an affixing means for affixing the visual status indicator along a portion of the patient circuit, and a light source for providing at said portion a light signal in dependence on a measured status of the fluid transported through the patient circuit.

WO2016054097 discloses an overhead passenger service unit (PSU) for a vehicle having a mounting mechanism for mounting the PSU above at least one vehicle seat; a dynamic seat row marker that provides an indication of a seat position and a status portion indicating a status of a passenger or trip aspect that is readily viewable from a vehicle aisle and is changeable during a trip; and a programmable active display that is readily viewable from a passenger seat and provides trip changeable information about the trip to the passenger.

### SUMMARY OF THE PREFERRED EMBODIMENTS

In accordance with a first aspect of the present invention there is provided an oxygen assembly according to claim 1.

In accordance with another aspect of the present invention there is provided vehicle oxygen indication system according to claim 2.

Preferred embodiments are provided in the dependent claims.

The present invention is a passenger oxygen system for aircraft used in case of cabin decompression with a visible flow indicator on the oxygen hose, on the passenger service unit, on a handheld device (e.g., a tablet, mobile phone or display dedicated to the oxygen indicator system) and/or on a central display available to the cabin crew from their current or seated position. The objective of the invention is to inform the passenger and/or the cabin crew whether oxygen is being delivered to the passenger oxygen mask or not. The passenger oxygen system includes an electric/electronic switch on the oxygen hose, on the passenger service unit and/or on a central display available to the cabin crew from their seated position. The system also integrates electrical wires in the hose to transmit the electricity for the flow indicator. The passenger oxygen system incorporates an electric/electronic switch, a light source and a hose integrating electrical wires which transmit and monitor the flow indication to the passenger service unit and/or a cabin management system accessible to cabin crew both when the crew is seated or when moving about the cabin.

In use, the flow of oxygen leads to a change of the state of the switch (e.g., the switch opens a valve so oxygen can flow through the hose and to the mask), which may include a change of the status of the light source or indicator. The status of the light source can be transmitted and monitored to the cabin management system. The integration of a light source to the passenger oxygen mask, on the oxygen hose, on the passenger service unit and/or on a central display available to the cabin crew from their seated position, improves the visibility of the flow indicator for the passenger and the cabin crew. Moreover, transmitting the data related to the oxygen flow monitoring to the cabin management system allows the cabin crew to identify from their seated position the passengers who may not be receiving oxygen. A further advantage of the invention is the identification of an oxygen supply that has accidentally delivered oxygen and therefore is not functional anymore.

Any type of electrical switch is within the scope of the present invention provided it can be switched between a closed and opened state. For example, a reed switch or a pressure switch can be used. Further, any type of light source can be used, including, but not limited to, light emitting diodes. Further, the oxygen system may be either gaseous or chemical.

It will be appreciated that the present invention can be used in scenarios other than an aircraft. For example the invention can be used in other modes of transportation including but not limited to trains, cars, buses and vertical take-off and landing aircraft.

Where an optional feature has been discussed above in relation to one aspect of the invention, it may, where appropriate, be applied to another aspect of the invention.

### BRIEF DESCRIPTION OF THE DRAWINGS

The invention may be more readily understood by referring to the accompanying drawings in which:
FIG. 1 is a schematic view of an oxygen indication system in accordance with a preferred embodiment of the present invention;
FIG. 2 is a cross-section of the hose of the oxygen indication system; and
FIG. 3 is a cross-section of the electrical switch of the oxygen indication system.

Like numerals refer to like parts throughout the several views of the drawings.

### DETAILED DESCRIPTION OF THE PREFERRED EMBODIMENTS

The following description and drawings are illustrative and are not to be construed as limiting. Numerous specific details are described to provide a thorough understanding of the disclosure. However, in certain instances, well-known or conventional details are not described in order to avoid obscuring the description. References to one or an embodiment in the present disclosure can be, but not necessarily are references to the same embodiment; and, such references mean at least one of the embodiments.

Reference in this specification to "one embodiment" or "an embodiment" means that a particular feature, structure, or characteristic described in connection with the embodiment is included in at least one embodiment of the-disclosure. The appearances of the phrase "in one embodiment" in various places in the specification are not necessarily all referring to the same embodiment, nor are separate or alternative embodiments mutually exclusive of other embodiments. Moreover, various features are described which may be exhibited by some embodiments and not by others. Similarly, various requirements are described which may be requirements for some embodiments but not other embodiments.

The terms used in this specification generally have their ordinary meanings in the art, within the context of the disclosure, and in the specific context where each term is used. Certain terms that are used to describe the disclosure are discussed below, or elsewhere in the specification, to provide additional guidance to the practitioner regarding the description of the disclosure. For convenience, certain terms may be highlighted, for example using italics and/or quotation marks: The use of highlighting has no influence on the scope and meaning of a term; the scope and meaning of a term is the same, in the same context, whether or not it is highlighted.

It will be appreciated that the same thing can be said in more than one way. Consequently, alternative language and synonyms may be used for any one or more of the terms discussed herein. No special significance is to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and is not intended to further limit the scope and meaning of the disclosure or of any exemplified term. Likewise, the disclosure is not limited to various embodiments given in this specification.

Without intent to further limit the scope of the disclosure, examples of instruments, apparatus, methods and their related results according to the embodiments of the present disclosure are given below. Note that titles or subtitles may be used in the examples for convenience of a reader, which in no way should limit the scope of the disclosure. Unless otherwise defined, all technical and scientific terms used herein have the same meaning as commonly understood by one of ordinary skill in the art to which this disclosure pertains. In the case of conflict, the present document, including definitions, will control.

It will be appreciated that terms such as "front," "back," "top," "bottom," "side," "short," "long," "up," "down," "aft," "forward," "inboard," "outboard" and "below" used herein are merely for ease of description and refer to the orientation of the components as shown in the figures. It should be understood that any orientation of the components described herein is within the scope of the present invention.

Referring now to the drawings, which are for purposes of illustrating the present invention and not for purposes of limiting the same, the drawings show a vehicle oxygen indication system 10. In a preferred embodiment, the oxygen indication system is used in an aircraft. However, this is not a limitation on the present invention.

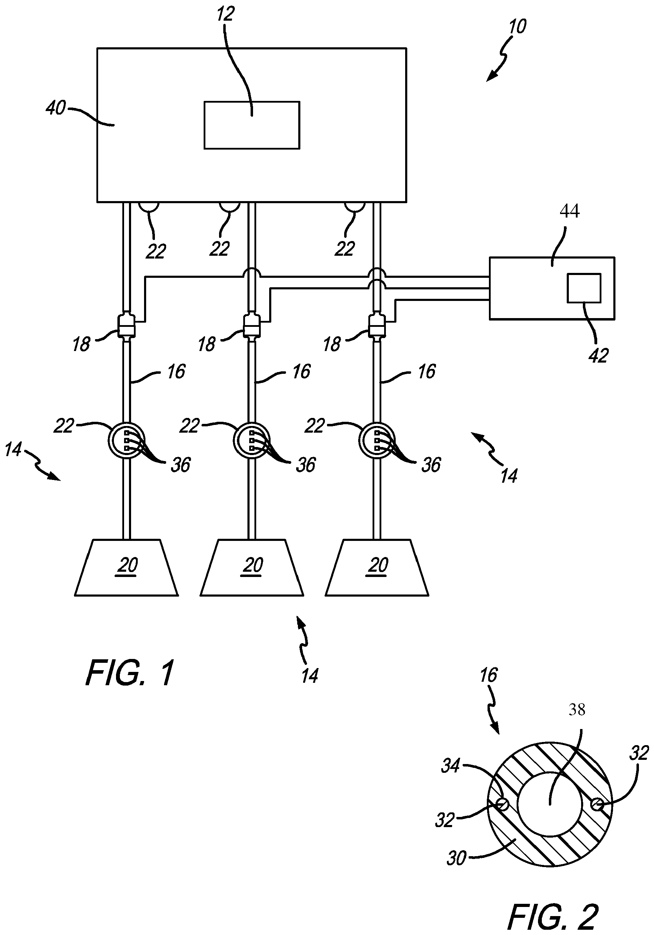

As shown in FIG. 1, in a preferred embodiment, the oxygen indication system 10 generally includes at least one oxygen source 12, a plurality of oxygen assemblies 14 and a power source 44. It will be appreciated that a single oxygen source and single power source can be used for all of the oxygen assemblies 14 or that multiple oxygen sources and power sources can be used. Each oxygen assembly 14 includes a hose 16, a switch 18, a mask 20 and a flow indicator 22 associated therewith. The hose includes a lumen 38 (see FIG. 2) through which oxygen can flow from the oxygen source 12 to the mask 20.

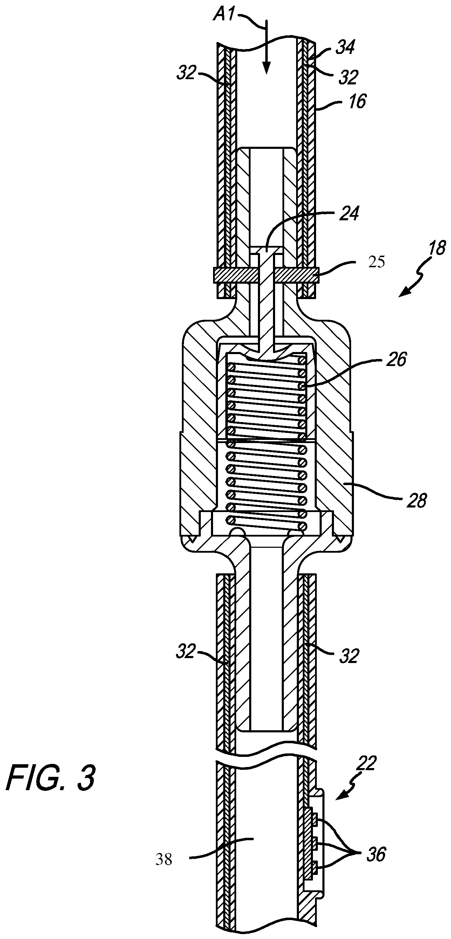

In a preferred embodiment, the switch 18 is switchable between a normal state where oxygen does not flow through the hose 16 and to the mask 20 and a flow state where oxygen flows through the hose 16 to the mask 20. FIG. 3 shows an exemplary switch 18 that closes the electrical circuit (so electricity flows to the flow indicator 22) when oxygen is flowing. The switch 18 includes a conductive plate 25, a plunger 24, and a return spring 26 that are housed in a housing 28. In use, when oxygen begins to flow through the hose 16 (see arrow A1 in FIG. 3), the plunger 24 (which is normally in the position shown in FIG. 3) is pushed into contact with the conductive plate 25, which closes the electrical circuit and, switches the switch from the normal state to the flow state, and switches or changes the flow indicator 22 from the static state to the indication state. In other words, in an embodiment where the flow indicator is a light, the flow indicator 22 lights up to indicate that oxygen is flowing to the mask.

As shown in FIGS. 2-3, in a preferred embodiment, the hose 16 includes a hose wall 30 through which one or more electrical lead wires 32 are embedded and extend (through a tunnel 34). This allows power to be routed to the switch 18 and/or the flow indicator 22. FIG. 3 shows the flow indicator 22 including a plurality of LEDs 36 mounted on the hose 16.

As shown in FIG. 1, in a preferred embodiment, the flow indicators 22 can be located on a personal service unit (PSU) 40. It will be appreciated that each oxygen assembly 14 is associated with a seat in the vehicle. Each seat may have its own PSU or a single PSU may service a plurality of seats. FIG. 1 shows a single PSU 40 with three flow indicators 22 mounted thereon that are each associated with a single oxygen assembly 14 (and seat). The PSU mounted flow indicator 22 is communicated with the switch 18 and is switched to the indication state when oxygen is flowing to the mask. It will be appreciated that the system can include one or more of any of the flow indicator positions (hose mounted, PSU mounted, display, etc.).

In another embodiment, the system 10 includes a control unit 42, that communicates the status of the oxygen assemblies 14 to one or more of a central display or mobile devices(s). This can be done wirelessly or through a wired system. For example, the system 10 can include Bluetooth or other wireless protocol so that the switch 18 can communicate its state as necessary.

Unless the context clearly requires otherwise, throughout the description and the claims, the words "comprise," "comprising," and the like are to be construed in an inclusive sense, as opposed to an exclusive or exhaustive sense; that is to say, in the sense of "including, but not limited to." As used herein, the terms "connected," "coupled," or any variant thereof, means any connection or coupling, either direct or indirect, between two or more elements; the coupling of connection between the elements can be physical, logical, or a combination thereof. Additionally, the words "herein," "above," "below," and words of similar import, when used in this application, shall refer to this application as a whole and not to any particular portions of this application. Where the context permits, words in the above Detailed Description of the Preferred Embodiments using the singular or plural number may also include the plural or singular number respectively. The word "or" in reference to a list of two or more items, covers all of the following interpretations of the word: any of the items in the list, all of the items in the list, and any combination of the items in the list.

The above-detailed description of embodiments of the disclosure is not intended to be exhaustive or to limit the teachings to the precise form disclosed above. While specific embodiments of and examples for the disclosure are described above for illustrative purposes, various modifications are possible ... as far as they remain within the scope of the appended claims, as those skilled in the relevant art will recognize. Further, any specific numbers noted herein are only examples: alternative implementations may employ differing values, measurements or ranges.

The teachings of the disclosure provided herein can be applied to other systems, not necessarily the system described above. The elements and acts of the various embodiments described above can be combined to provide further embodiments. Any measurements described or used herein are merely exemplary and not a limitation on the present invention. Other measurements can be used. Further, any specific materials noted herein are only examples: alternative implementations may employ differing materials.

Aspects of the disclosure can be modified, if necessary, to employ the systems, functions, and concepts of the various references described above to provide yet further embodiments of the disclosure.

These and other changes can be made to the disclosure in light of the above Detailed Description of the Preferred Embodiments. While the above description describes certain embodiments of the disclosure, and describes the best mode contemplated, no matter how detailed the above appears in text, the teachings can be practiced in many ways. Details of the system may vary considerably in its implementation details, while still being encompassed by the subject matter disclosed herein. As noted above, particular terminology used when describing certain features or aspects of the disclosure should not be taken to imply that the terminology is being redefined herein to be restricted to any specific characteristics, features or aspects of the disclosure with which that terminology is associated.

Accordingly, although exemplary embodiments of the invention have been shown and described, it is to be understood that all the terms used herein are descriptive rather than limiting, and that many changes, modifications, and substitutions may be made by one having ordinary skill in the art without departing from the scope of the invention as defined by the claims.

## Claims

1. An oxygen assembly (14) comprising:
a hose (16) configured to be in flow communication with an oxygen source (12), wherein the hose (16) includes a hose wall (30),
a switch (18) positioned on the hose, wherein an electric lead wire (32) is disposed in the hose wall (30) and is configured to electrically communicate the switch (18) with a power source (44), wherein the switch (18) includes a plunger (24),
a mask (20) positioned at a distal end of the hose (16), and
a flow indicator (22), wherein the flow indicator (22) is a light (36) that is positioned on the hose (16), wherein the electric lead wire (32) is configured to electrically communicate the light with the power source (44),
wherein the hose (16) includes a lumen (38) through which oxygen can flow from the oxygen source (12) to the mask (20), wherein the switch (18) is switchable between a normal state when oxygen is not flowing from the oxygen source (12) to the mask (20) and a flow state where oxygen flows to the mask (20),
wherein when the switch (18) is in the flow state, the light (36) is switched from a static state to an indication state, wherein when the oxygen flows from the oxygen source (12) to the mask (20), the plunger (24) closes an electrical circuit thereby switching the light (36) from the static state to the indication state.

2. A vehicle oxygen indication system comprising:
at least one oxygen source (12), and
a plurality of oxygen assemblies (14) according to claim 1.

3. The vehicle oxygen indication system of claim 2 wherein a second flow indicator (22) is located on a personal service unit (40) associated with a seat.

4. The vehicle oxygen indication system of claim 2 or 3 further comprising a control unit (42), wherein the switch (18) of each oxygen assembly is wirelessly in communication with the control unit (42), and wherein a third flow indicator is located on a display.

5. The vehicle oxygen indication system of claim 4 wherein the display is mounted in an interior of the vehicle.

6. The vehicle oxygen indication system of claim 4 wherein the display is part of a mobile device.

## Patentansprüche

1. Sauerstoffaggregat (14), das Folgendes umfasst:
einen Schlauch (16), der dafür konfiguriert ist, in Durchflussverbindung mit einer Sauerstoffquelle (12) zu stehen, wobei der Schlauch (16) eine Schlauchwand (30) einschließt,
einen Schalter (18), der an dem Schlauch positioniert ist, wobei ein elektrischer Leitungsdraht (32) in der Schlauchwand (30) angeordnet ist und dafür konfiguriert ist, den Schalter (18) elektrisch mit einer Energiequelle (44) zu verbinden, wobei der Schalter (18) einen Kolben (24) einschließt,
eine Maske (20), die an einem distalen Ende des Schlauchs (16) positioniert ist, und
einen Durchflussanzeiger (22), wobei der Durchflussanzeiger (22) eine Leuchte (36) ist, die an dem Schlauch (16) positioniert ist, wobei der elektrische Leitungsdraht (32) dafür konfiguriert ist, die Leuchte elektrisch mit der Energiequelle (44) zu verbinden,
wobei der Schlauch (16) ein Lumen (38) einschließt, durch das Sauerstoff von der Sauerstoffquelle (12) zu der Maske (20) strömen kann, wobei der Schalter (18) umschaltbar ist zwischen einem normalen Zustand, wenn kein Sauerstoff von der Sauerstoffquelle (12) zu der Maske (20) strömt, und einem Durchflusszustand, wenn Sauerstoff zu der Maske (20) strömt,
wobei, wenn sich der Schalter (18) in dem Durchflusszustand befindet, die Leuchte (36) von einem statischen Zustand zu einem Anzeigezustand umgeschaltet wird, wobei, wenn der Sauerstoff von der Sauerstoffquelle (12) zu der Maske (20) strömt, der Kolben (24) einen elektrischen Stromkreis schließt, wodurch die Leuchte (36) von dem statischen Zustand zu dem Anzeigezustand umgeschaltet wird.

2. Fahrzeug-Sauerstoffanzeigesystem, das Folgendes umfasst:
mindestens eine Sauerstoffquelle (12) und
eine Vielzahl von Sauerstoffaggregaten (14) nach Anspruch 1.

3. Fahrzeug-Sauerstoffanzeigesystem nach Anspruch 2, wobei sich ein zweiter Durchflussanzeiger (22) an einer persönlichen Bedienungseinheit (40) befindet, die mit einem Sitz verknüpft ist.

4. Fahrzeug-Sauerstoffanzeigesystem nach Anspruch 2 oder 3, das ferner eine Steuereinheit (42) umfasst, wobei der Schalter (18) jedes Sauerstoffaggregats drahtlos in Verbindung mit der Steuereinheit (42) steht und wobei sich ein dritter Durchflussanzeiger an einer Anzeige befindet.

5. Fahrzeug-Sauerstoffanzeigesystem nach Anspruch 4, wobei die Anzeige in einem Inneren des Fahrzeugs angebracht ist.

6. Fahrzeug-Sauerstoffanzeigesystem nach Anspruch 4, wobei die Anzeige Teil eines mobilen Geräts ist.

## Revendications

1. Ensemble d'oxygène (14) comprenant :
un tuyau (16) configuré pour être en communication fluidique avec une source d'oxygène (12), dans lequel le tuyau (16) inclut une paroi de tuyau (30),
un commutateur (18) positionné sur le tuyau, dans lequel un fil électrique (32) est disposé dans la paroi de tuyau (30) et est configuré pour faire communiquer électriquement le commutateur (18) avec une source d'alimentation (44), dans lequel le commutateur (18) inclut un piston (24),
un masque (20) positionné à une extrémité distale du tuyau (16), et
un indicateur de débit (22), dans lequel l'indicateur de débit (22) est un témoin (36) qui est positionné sur le tuyau (16), dans lequel le fil électrique (32) est configuré pour faire communiquer électriquement le témoin avec la source d'alimentation (44),
dans lequel le tuyau (16) inclut une lumière (38) à travers laquelle l'oxygène peut s'écouler de la source d'oxygène (12) au masque (20), dans lequel le commutateur (18) est commutable entre un état normal où l'oxygène ne s'écoule pas de la source d'oxygène (12) vers le masque (20) et un état d'écoulement où l'oxygène s'écoule vers le masque (20),
dans lequel, quand le commutateur (18) est dans l'état d'écoulement, le témoin (36) est commuté d'un état statique à un état d'indication, dans lequel, quand l'oxygène s'écoule de la source d'oxygène (12) vers le masque (20), le piston (24) ferme un circuit électrique en commutant ainsi le témoin (36) de l'état statique à l'état d'indication.

2. Système d'indication d'oxygène d'un véhicule comprenant :
au moins une source d'oxygène (12), et
une pluralité d'ensembles d'oxygène (14) selon la revendication 1.

3. Système d'indication d'oxygène d'un véhicule selon la revendication 2, dans lequel un deuxième indicateur de débit (22) est situé sur une unité de service personnelle (40) associée à un logement.

4. Système d'indication d'oxygène d'un véhicule selon la revendication 2 ou 3, comprenant en outre une unité de commande (42), dans lequel le commutateur (18) de chaque ensemble d'oxygène est en communication sans fil avec l'unité de commande (42), et dans lequel un troisième indicateur de débit est situé sur un écran.

5. Système d'indication d'oxygène d'un véhicule selon la revendication 4, dans lequel l'écran est monté à l'intérieur du véhicule.

6. Système d'indication d'oxygène d'un véhicule selon la revendication 4, dans lequel l'écran fait partie d'un dispositif mobile.
